# EUROPÄISCHE PATENTSCHRIFT

(11) **EP 1 220 661 B1**
(45) Veröffentlichungstag und Bekanntmachung des Hinweises auf die Patenterteilung: **16.11.2005**
(21) Anmeldenummer: 00966096.0
(22) Anmeldetag: 30.09.2000
(51) Int. Cl.: A61K 9/70, A61K 31/60, A61K 47/44, A61K 47/22

(54) **TRANSDERMALES THERAPEUTISCHES SYSTEM ZUR ABGABE VON ACETYLSALICYLSÄURE UND/ODER SALICYLSÄURE**
TRANSDERMAL THERAPEUTIC SYSTEM FOR ADMINISTERING ACETYLSALICYLIC ACID AND/OR SALICYLIC ACID
SYSTEME THERAPEUTIQUE TRANSDERMIQUE PERMETTANT DE DELIVRER DE L'ACIDE ACETYLSALICYLIQUE ET/OU DE L'ACIDE SALICYLIQUE

(30) Priorität: 13.10.1999 DE 19949202
(43) Veröffentlichungstag der Anmeldung: 10.07.2002
(73) Patentinhaber: LTS LOHMANN Therapie-Systeme AG, 56626 Andernach (DE)
(72) Erfinder: FRANKE, Hanshermann, 22889 Tangstedt / Wilstedt-Siedlung (DE); KINDEL, Heinrich, 56581 Ehlscheid (DE); HOFFMANN, Gerd, 56566 Neuwied (DE)
(74) Vertreter: Flaccus, Rolf-Dieter
(86) Internationale Anmeldenummer: PCT/EP2000/009617
(87) Internationale Veröffentlichungsnummer: WO 2001/026637

(56) Entgegenhaltungen:
- EP-A- 0 356 382
- DE-A- 4 332 093
- DE-A- 19 701 059
- GB-A- 2 141 025

## Beschreibung

Die Erfindung betrifft ein transdermales therapeutisches System, welches die Abgabe von Acetylsalicylsäure oder Salicylsäure, oder beider Wirkstoffe zusammen, an die Haut ermöglicht. Sie betrifft des weiteren ein Verfahren zur Herstellung eines solchen Systems.

Der Wirkstoff Acetylsalicylsäure findet breite Anwendung als Schmerzmittel, fiebersenkendes Mittel, zur Entzündungshemmung und zur Hemmung der Thrombozytenaggregation. Acetylsalicylsäure greift in den Prostaglandin-Stoffwechsel ein und unterdrückt dadurch die Bildung von Mediatorstoffen, welche beim Schmerz- und Entzündungsgeschehen eine zentrale Rolle spielen. Dies wird durch Hemmung einer Cyclooxygenase bewirkt, eines Enzyms des Prostaglandin-Biosyntheseweges. Deshalb wird der Wirkstoff Acetylsalicylsäure zu der Gruppe der sogenannten COX-2-Inhibitoren gerechnet.
Salicylsäure wirkt ebenfalls entzündungshemmend, daneben wird sie auch aufgrund ihrer antiseptischen und gerinnungshemmenden Eigenschaften verwendet.

Bei der oralen Verabreichung von Acetylsalicylsäure oder Salicylsäure besteht, insbesondere bei längerandauernder Anwendung, das Risiko von gastrointestinalen Nebenwirkungen, beispielsweise in Form von Mikroblutungen. Dieser Nachteil läßt sich vermeiden, indem man eine Darreichungsform wählt, mittels derer der Wirkstoff dem Organismus unter Umgehung des Magen-DarmTraktes zugeführt werden kann. Die transdermale Verabreichung erscheint hierfür besonders geeignet. Voraussetzung ist jedoch, daß ein Applikationssystem existiert, welches in der Lage ist, den Wirkstoff Acetylsalicylsäure oder Salicylsäure über einen gewissen Zeitraum mit ausreichend hohen Fluxraten aus einem Reservoir an die Haut abzugeben.

Die Permeationsfähigkeit von Acetylsalicylsäure durch die menschliche Haut, und damit ihre grundsätzliche Eignung als Bestandteil von wirkstoffhaltigen Hautpflastern ist grundsätzlich bekannt (Journal of Pharmaceutical Science 176 (1987), pp. 451-454). Der dabei erzielte Wirkstoff-Flux entspricht aber im Hinblick auf den zeitlichen Eintritt und das Ausmaß der beabsichtigten pharmakologischen Wirkungen, beispielsweise hinsichtlich einer frühestmöglichen Inaktivierung der thrombozytenständigen Cyclooxygenase, nicht den gestellten Erwartungen.

Es ist im Stand der Technik zwar eine Vielzahl von Substanzen bekannt, welche die Penetration von Wirkstoffen durch die Haut unterstützen können ("Enhancer"). Die theoretischen Grundlagen für diese Enhancer-Wirkung sind größtenteils noch unbekannt; es existieren jedoch Ansätze, diese Wirkung anhand von Modellen zu erklären (Williams A. C. & Barry B. W., "Skin absorption enhancers", Crit. Rev. Ther. Drug Carrier Syst. (US) 1992, 9/3-4, 305-353)

Das Patentdokument DE 197 01 059 offenbart ein Acetylsalicylsäure enthaltendes transdermales therapeutisches System in Pflasterform, mit einer Rückschicht, einem damit ver bundenen Wirkstoffreservoir, einer bei Abwesenheit anderer Steuermechanismen die Wirkstoffabgabe steuernden Membran, einer Haftklebe einrichtung zur Befestigung des Systems auf der Haut und einer vor der Applikation ablösbaren Schutzschicht, dadurch gekennzeichnet, dass es Limonen enthält.

Das Patentdokument EP-A-0 356 382 offenbart ein Acetylsalicylsäure enthaltendes transdermales therapeutisches System in Pflasterform, dadurch gekennzeichnet, dass es 2-Pyrrolidon enthält.

Aufgabe der vorliegenden Erfindung war es deshalb, eine transdermale Darreichungsform für die Wirkstoffe Acetylsalicylsäure und Salicylsäure bereitzustellen, welche einen genügend hohen Wirkstoff-Flux in vivo ermöglicht, und welche mittels gängiger Herstellungsverfahren kostengünstig produziert werden kann.

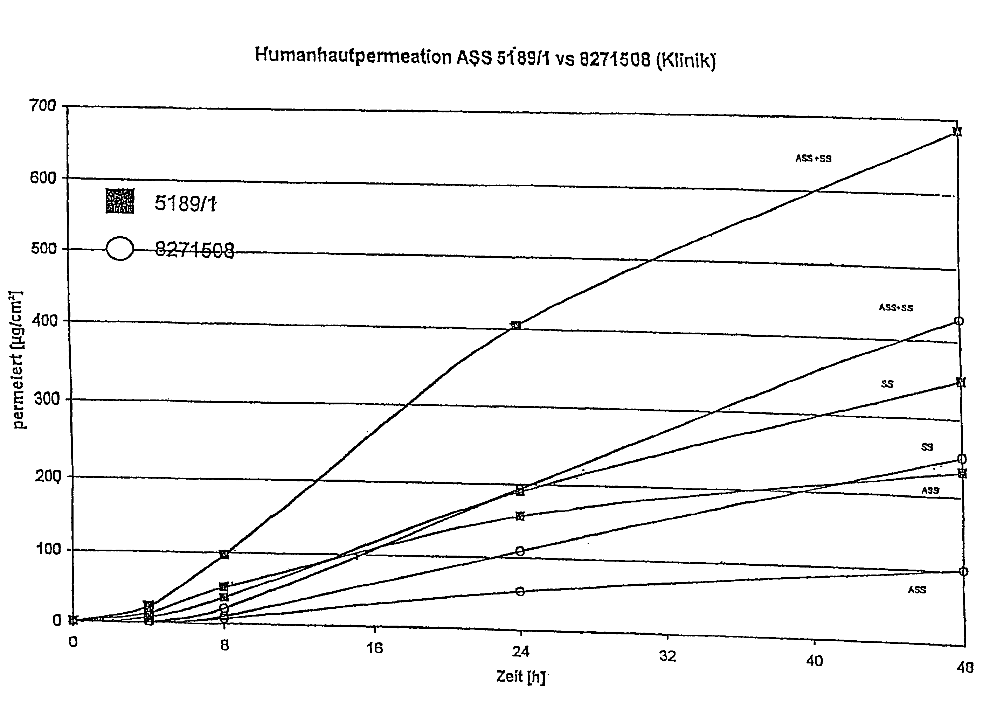

Überraschenderweise wird die Aufgabe gelöst durch ein transdermales therapeutisches System (TTS) in Pflasterform gemäß Anspruch 1. Das erfindungsgemäße TTS ist dadurch gekennzeichnet, daß es mindestens einen Bestandteil aus der Gruppe der Pyrrol-Derivate und mindestens einen Bestandteil aus der Gruppe der Terpene enthält. Die gemeinsame Anwesenheit von Bestandteilen aus den beiden Gruppen führt zu einer unerwarteten Steigerung des in vivo-Wirkstofftransports durch die Haut (vgl. Tab. 1 und Fig. 1).

Als Vertreter aus der Gruppe der Pyrrol-Derivate hat sich 2-Pyrrolidon als besonders wirksam erwiesen.

Als Vertreter aus der Gruppe der Terpene eignet sich Limonen, jedoch können mit Vorteil auch natürliche Terpengemische eingesetzt werden, deren Hauptbestandteil Limonen ist, z. B. Zitronenöl (Oleum citri).

Um einen optimalen Wirkstoff-Flux zu erzielen, hat es sich als besonders vorteilhaft erwiesen, wenn die Bestandteile aus der Gruppe der Pyrrol-Derivate in einem Konzentrationsbereich von 0,1-15 %, vorzugsweise von 2 bis 10 %, und die Bestandteile aus der Gruppe der Terpene in einem Konzentrationsbereich von 1-30 %, vorzugsweise von 10-20 % eingesetzt werden, jeweils bezogen auf die Gesamtmasse der Matrix.

Die erfindungsgemäßen TTS weisen vorzugsweise einen schichtartigen Aufbau mit mindestens einer Polymer-Matrixschicht auf. Diese kann zugleich die Funktion des Wirkstoffreservoirs ausüben. Im Einzelfall kann es auch von Vorteil sein, wenn die erfindungsgemäßen TTS zwei oder mehrere Matrixschichten aufweisen. Diese können unterschiedliche Wirkstoffe oder unterschiedliche Wirkstoffkonzentrationen aufweisen. Ferner können sich die einzelnen Matrixschichten auch bezüglich ihres Gehalts an Enhancern oder anderen Zusatzstoffen unterscheiden.

Die Matrixschicht kann zudem auch haftklebend ausgebildet sein, um die Befestigung des Pflasters auf der Haut zu ermöglichen. Falls das wirkstoffhaltige Reservoir selbst nicht oder nicht ausreichend haftklebend ist, kann es mit einer speziellen Haftklebeeinrichtung verbunden werden, die einen dauernden Kontakt des Systems zur Haut sicherstellt. Hierfür kommen grundsätzlich die gleichen Materialien in Frage wie für die Polymermatrix des Wirkstoffreservoirs.

Um eine Steuerung der Freisetzung zu ermöglichen, wenn dies nicht durch andere Mechanismen bewirkt wird, kann das Reservoir mit einer Steuermembran versehen werden, welche die Abgabe des Wirkstoffs an die Haut steuert.

Vorzugsweise sind die Polymer-Matrixschichten der erfindungsgemäßen TTS aus Polymeren aufgebaut, welche aus der Gruppe ausgewählt sind, die Polyacrylsäureester und deren Copolymere, Polymere auf (Meth)Acrylatbasis, Ethylenvinylacetat-Copolymere, Polyisobutylene, Polyterpene, Cellulosederivate, Kautschuke, Synthesekautschuke und Heißschmelzkleber umfaßt. Als Polymere auf Acrylatbasis werden Copolymere aus 2-Ethylhexylacrylat, Vinylacetat und Acrylsäure mit oder ohne Vernetzer bevorzugt. Als Poly-Methacrylate werden vorrangig Copolymere auf der Basis von Dimethylaminomethacrylat und neutralen Methacrylsäureestern verwendet. Als Synthesekautschuke werden bevorzugt Blockcopolymere auf der Basis von Styrol und 1,3-Dienen, z. B. lineare Styrol-Isopren-Blockpolymere eingesetzt. Auch Polymergemische können zum Einsatz kommen.

Bei einer besonders bevorzugten Ausführungsform der Erfindung enthält mindestens eine Matrixschicht Polymerbestandteile, welche aus der Gruppe der substituierten Cellulosen, vorzugsweise der Methyl- oder Ethylcellulosen ausgewählt sind. Bei einer weiteren bevorzugten Ausführungsform ist vorgesehen, daß mindestens eine Matrixschicht Ester des hydrierten Kolophoniums enthält, vorzugsweise dessen Methyl- oder Glycerinester. Durch die Auswahl der Matrix-Polymere und der sonstigen Matrixbestandteile können die mechanischen Eigenschaften der TTS, wie Kohäsion und Klebrigkeit / Tack beeinflußt werden.

Der schichtartige Aufbau der erfindungsgemäßen TTS umfaßt gemäß dem Oberbegriff des Hauptanspruchs außerdem eine undurchlässige Rückschicht sowie eine ablösbare Schutzschicht. Als Rückschicht eignen sich vor allem Polyester, die sich durch besondere Festigkeit und Diffusionsfestigkeit auszeichnen, darüber hinaus aber nahezu beliebige andere hautverträgliche Kunststoffe, wie z. B. Polyvinylchlorid, Ethylenvinylacetat, Vinylacetat, Polyethylen, Polypropylen, Cellulosederivate und viele andere mehr. Im Einzelfall kann die Rückschicht mit einer zusätzlichen Auflage versehen werden, z. B. durch Bedampfung mit Metallen oder anderen diffusionssperrenden Zusatzstoffen wie Siliciumdioxid, Aluminiumoxid oder ähnlicher, dem Fachmann bekannter Stoffe. Für die ablösbare Schutzschicht können dieselben Materialien verwendet werden wie für die Rückschicht, vorausgesetzt, daß sie durch eine geeignete Oberflächenbehandlung wie z. B. Silikonisierung ablösbar gemacht wurden. Es können aber auch andere ablösbare Schutzschichten wie Polytetrafluorethylen-behandeltes Papier, Cellophan, Polyvinylchlorid oder ähnliche verwendet werden.

Um die Wirkung der erfindungsgemäßen TTS zu verbessern oder um diese an unterschiedliche Anforderungen anzupassen, können noch weitere Hilfs- oder Zusatzstoffe hinzugefügt werden. Dabei kommen vor allem Weichmacher und Permeationsbeschleuniger in Frage. Daneben können auch Klebrigmacher, Stabilisatoren, Füllstoffe und Trägerstoffe zugesetzt werden. Die hierfür in Frage kommenden pharmazeutisch unbedenklichen Zusätze sind dem Fachmann bekannt.

Als Weichmacher werden bevorzugt Verbindungen aus der Gruppe der Kohlenwasserstoffe, Alkohole, Carbonsäuren und ihren Derivaten, Ether, Ester oder Amine eingesetzt, wobei auch einzelne Weichmacherverbindungen miteinander kombiniert eingesetzt werden können. Eine bevorzugte Ausführungsform der Erfindung sieht einen Weichmacher-Gehalt 0-30 %, vorzugsweise von 5-20 % vor, jeweils bezogen auf die Gesamtmasse der Matrix.

Eine weitere bevorzugte Ausführungsform ist dadurch gekennzeichnet, daß die TTS ein natürliches oder partialsynthetisches Triglycerid oder eine Mischung dieser Triglyceride in einem Konzentrationsbereich von 0-30 %, vorzugsweise im Bereich von 5-20 % enthalten, bezogen auf die Gesamtmasse der Matrix.

Eine weitere Steigerung der Wirkstoffabgabe wird durch die im Unteranspruch 13 beschriebene Ausführungsvariante ermöglicht, welche vorsieht, daß die TTS Permeationsbeschleuniger aus der Gruppe der Polyoxyethylen-Derivate und nicht-ionogenen Tenside, vorzugsweise der Sorbitanfettsäureester, enthalten. Die Konzentration des oder der Permeationsbeschleuniger liegt in einem Bereich von 0-5 %, vorzugsweise von 1-3 %, bezogen auf die Gesamtmasse der Matrix.

Für gewöhnlich liegt der Wirkstoff Acetylsalicylsäure oder Salicylsäure dispergiert oder gelöst in einer oder mehreren Polymermatrixschichten der erfindungsgemäßen TTS in möglichst homogener Verteilung vor. Gemäß einer besonderen Ausführungsform kann der Wirkstoff aber auch in einem beutelförmigen Reservoir vorliegen, welches mit einer flüssigen, hochviskosen, halbfesten oder thixotropen Matrix gefüllt ist, die den Wirkstoff enthält. Besonders vorteilhaft ist es, wenn das halbfeste oder thixotrope Wirkstoffreservoir einen Gelbildner enthält. Die der Haut abgewandte Beutelrückseite muß dabei wirkstoffundurchlässig, die der Haut zugewandte Seite wirkstoffdurchlässig sein. Optional kann eine wirkstoffdurchlässige Membran die Steuerung der Wirkstofffreisetzung übernehmen.

Um eine möglichst gute Freisetzungsrate zu ermöglichen, wird vorzugsweise eine möglichst hohe Wirkstoffkonzentration in den wirkstoffhaltigen Schichten angestrebt, wobei allerdings zu beachten ist, daß bei zu hohen Konzentrationen die Stabilität des Systems beeinträchtigt werden kann. Bei den erfindungsgemäßen TTS werden deshalb Wirkstoffkonzentrationen im Bereich von 5-75 %, insbesondere von 15-45 % bevorzugt, bezogen auf die Gesamtmasse der wirkstoffhaltigen Schichten.

Nachfolgend wird die Erfindung anhand von Beispielen näher erläutert.

Die Steigerung der Freisetzung von Acetylsalicylsäure und/oder Salicylsäure bei den erfindungsgemäßen TTS wird durch die Meßwerte in Tabelle 1 und deren graphische Darstellung in Fig. 1 veranschaulicht. Daraus ist ersichtlich, daß bei TTS, welche erfindungsgemäß eine Kombination aus 2-Pyrrolidon und einem Terpengemisch enthalten, stets eine deutliche Steigerung bei der Humanhautpenetration zu verzeichnen war, und zwar sowohl bei Verwendung nur jeweils eines Wirkstoffs (ASS oder SS), oder beider Wirkstoffe in Kombination.

Die bei der Bestimmung der Humanhautpenetration eingesetzten TTS wurden wie folgt hergestellt:
1) Zunächst wurden 2-Pyrrolidon und ASS in einem Polyacrylatkleber dispergiert. Es entstand eine Suspension, die auf eine 100 µm-PET-Folie (release liner/ Schutzschicht) beschichtet wurde. Nach Trocknung wurde dieses Laminat mit einer HDPE-Folie (intermediate liner) abgedeckt, aufgerollt und vor Feuchtigkeit geschützt gelagert. Dieses getrocknete Laminat wird als Laminatstrich 1 bezeichnet.
2) Oleum citri wurde mit einem Polymethacrylat (Plastoid® B) zu einer homogenen viskosen Lösung verarbeitet und auf eine 19 µm-PET-Folie (backing layer /Rückschicht) beschichtet, woraus Laminatstrich 2 resultiert (feucht).
3) Der Laminatstrich 1 wurde auf den noch feuchten Laminatstrich 2 kaschiert. Das resultierende 2-Schichtlaminat wurde zu einer Breitrolle aufgewickelt. Dieses wurde dann zu Schmalrollen aufgeschnitten, aus welchen wiederum ASS-TTS vereinzelt wurden.

Als Vergleichsbeispiel (Nr. 8271508) diente eine entsprechende Formulierung ohne 2-Pyrrolidon.

Die Bestimmung der Permeationsraten durch Humanhaut erfolgte mit Hilfe von Franz-Diffusionszellen.

Die Ergebnisse der Permeationsversuche sind in Tabelle 1 zusammengefaßt. Dabei beziehen sich die mit "SS", "ASS" bzw. "ASS Sum" bezeichneten Zeilen auf Freisetzungs-Werte, die mit TTS erhalten wurden, welche entweder Salicylsäure oder Acetylsalicylsäure, oder Acetylsalicylsäure in Kombination mit Salicylsäure enthielten.
Die in den Tabellenspalten enthaltenen Werte geben die pro Flächeneinheit (cm²) freigesetzte Wirkstoffmenge (in µg) wieder.

Figur 1 zeigt eine graphische Darstellung der Daten aus Tabelle 1.

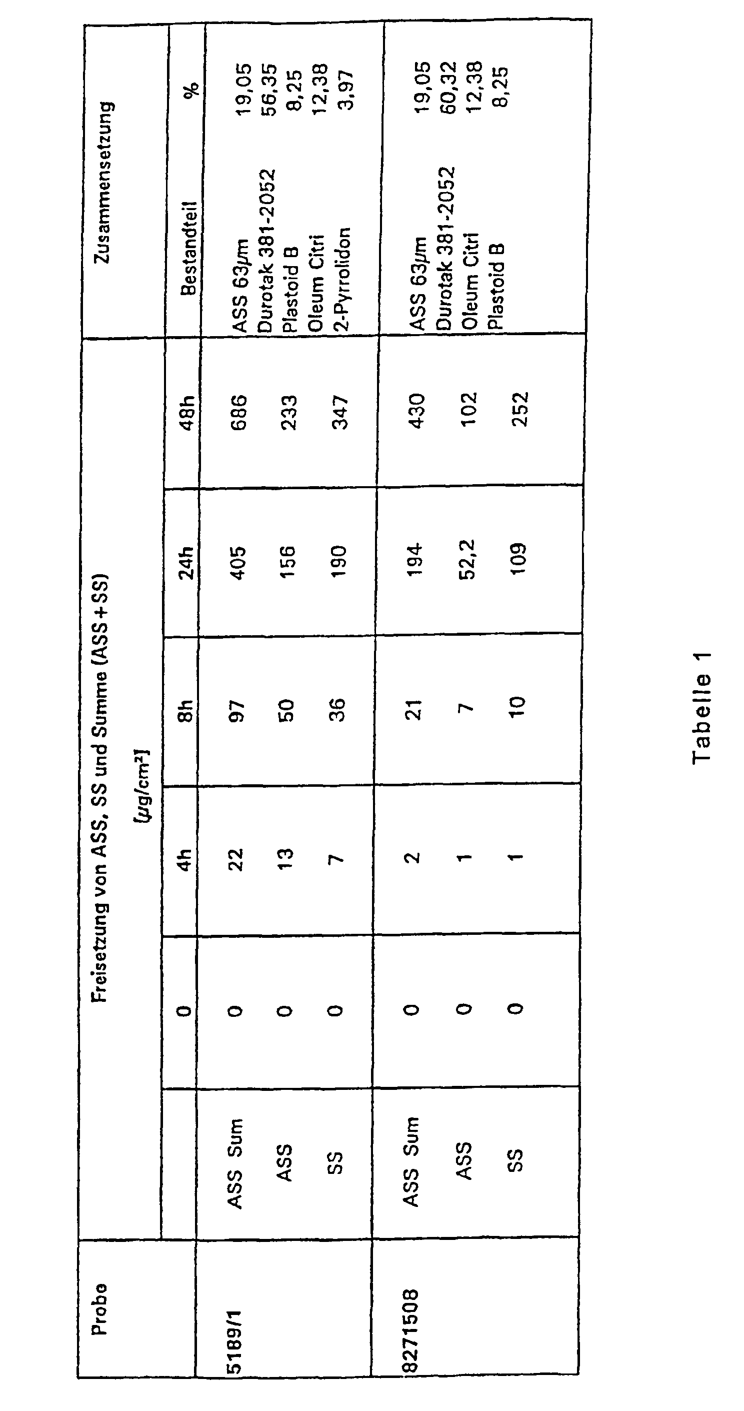

## Patentansprüche

1. Acetylsalicylsäure und/oder Salicylsäure enthaltendes transdermales therapeutisches System in Pflasterform, mit einer Rückschicht, einem damit verbundenen Wirkstoffreservoir, einer bei Abwesenheit anderer Steuermechanismen die Wirkstoffabgabe steuernden Membran, einer Haftklebe-Einrichtung zur Befestigung des Systems auf der Haut und einer vor der Applikation ablösbaren Schutzschicht, **dadurch gekennzeichnet, daß** es einen schichtförmigen Aufbau mit mindestens einer Polymer-Matrixschicht aufweist, wobei die Polymer-Matrixschicht(en) aus Polymeren aufgebaut ist/sind, welche aus der Polyacrylsäureester und deren Copolymere, Polymere auf (Meth)acrylatbasis, Polyacrylate, Polyisobutylene, Polyterpene, Ethylenvinylacetat-Copolymere, Heißschmelzkleber und substituierte Cellulosen, vorzugsweise Methyl- und Ethylcellulosen, und Mischungen dieser Polymere umfassenden Gruppe ausgewählt sind; und daß das transdermale therapeutische System
- einen Bestandteil aus der Gruppe der Pyrrol-Drivate, bei dem es sich um 2-Pyrrolidon handelt, und
- Limonen oder ein natürliches Terpengemisch enthält, dessen Hauptbestandteil Limonen ist.

2. Transdermales therapeutisches System nach Anspruch 1, **dadurch gekennzeichnet, daß** der Bestandteil aus der Gruppe der Pyrrolderivate in einem Konzentrationsbereich von 0,1-15 %, vorzugsweise von 2 bis 10 %, und Limonen oder das natürliche Terpengemisch in einem Konzentrationsbereich von 1-30 %, vorzugsweise von 10-20 %, eingesetzt werden, jeweils bezogen auf die Gesamtmasse der Matrix.

3. Transdermales therapeutisches System nach Anspruch 1 oder 2, **dadurch gekennzeichnet, daß** mindestens eine Matrixschicht Ester des hydrierten Kolophoniums enthält, vorzugsweise dessen Methyl-oder Glycerinester.

4. Transdermales therapeutisches System nach einem der vorangehenden Ansprüche, **dadurch gekennzeichnet, daß** es einen oder mehrere Weichmacher enthält, ausgewählt aus der Gruppe, welche Kohlenwasserstoffe, Alkohole, Carbonsäuren und ihre Derivate, Ether, Ester oder Amine enthält.

5. Transdermales therapeutisches System nach Anspruch 4, **dadurch gekennzeichnet, daß** es Weichmacher in einer Konzentration von 0-30 %, vorzugsweise von 5-20 % enthält, jeweils bezogen auf die Gesamtmasse der Matrix.

6. Transdermales therapeutisches System nach einem der vorangehenden Ansprüche, **dadurch gekennzeichnet, daß** es ein natürliches oder partialsynthetisches Triglycerid oder eine Mischung dieser Triglyceride in einem Konzentrationsbereich von 0-30 %, vorzugsweise im Bereich von 5-20 %, enthält, bezogen auf die Gesamtmasse der Matrix.

7. Transdermales therapeutisches System nach einem der vorangehenden Ansprüche, **dadurch gekennzeichnet, daß** es mindestens einen Permeationsbeschleuniger enthält, ausgewählt aus der Gruppe, welche Polyoxyethylen-Derivate und nicht-ionogene Tenside, vorzugsweise Sorbitanfettsäureester, umfaßt, wobei die Konzentration des oder der Permeationsbeschleuniger in einem Bereich von 0-5 %, vorzugsweise von 1-3 % liegt, bezogen auf die Gesamtmasse der Matrix.

8. Transdermales therapeutisches System nach einem der vorangehenden Ansprüche, **dadurch gekennzeichnet, daß** die Konzentration der Acetylsalicylsäure und/oder Salicylsäure insgesamt 5-75 %, vorzugsweise 15-45 % beträgt, bezogen auf die Gesamtmasse der wirkstoffhaltigen Schichten.

9. Acetylsalicylsäure und/oder Salicylsäure enthaltendes transdermales therapeutisches System in Pflasterform, mit einer Rückschicht, einem damit verbundenen Wirkstoffreservoir, einer bei Abwesenheit anderer Steuermechanismen die wirkstoffabgabe steuernden Membran, einer Haftklebe-Einrichtung zur Befestigung des Systems auf der Haut und einer vor der Applikation ablösbaren Schutzschicht, **dadurch gekennzeichnet, daß** es ein beutelförmiges, mit einer flüssigen, hochviskosen, halbfesten oder thixotropen Matrix gefülltes Wirkstoffreservoir besitzt, und
- einen Bestandteil aus der Gruppe der Pyrrol-Drivate, bei dem es sich um 2-Pyrrolidon handelt, und
- Limonen und oder ein natürliches Terpengemisch enthält, dessen Hauptbestandteil Limonen ist.

10. Transdermales therapeutisches System nach Anspruch 9, **dadurch gekennzeichnet, daß** das beutelförmige Wirkstoffreservoir einen Gelbildner enthält.

11. Verfahren zur Herstellung eines transdermalen therapeutischen Systems nach einem der Ansprüche 1 bis 8, **gekennzeichnet durch** folgende Schritte:
a) Herstellung einer ersten Polymer-Matrixschicht, die 2-Pyrrolidon und den genannten Wirkstoff enthält, **durch** Beschichtung einer Polyacrylatkleber, 2-Pyrrolidon und Acetylsalicylsäure und/oder Salicylsäure enthaltenden Suspension auf eine Folie, und nachfolgende Trocknung;
b) Herstellung einer zweiten Polymer-Matrixschicht, die Limonen enthält, **durch** Beschichtung einer Limonen oder ein natürliches Terpengemisch, dessen Hauptbestandteil Limonen ist, sowie Polymethacrylat enthaltenden Lösung auf eine Folie;
c) Bildung eines zumindest zweischichtigen Laminates **durch** Aufkaschieren der getrockneten ersten Polymer-Matrixschicht auf die noch feuchte zweite Polymer-Matrixschicht.

## Claims

1. Transdermal therapeutic system in form of a patch, containing acetylsalicylic acid and/or salicylic acid, with a back layer, an active substance reservoir connected therewith, a membrane controlling the release of active substance if no other control mechanisms are present, a pressure-sensitive adhesive device for fixing the system to the skin and a protective layer to be separated before application, **characterized in that** it has a layer-like structure with at least one polymer matrix layer, the polymer matrix layer(s) being composed of polymers chosen from the group comprising esters of polyacrylic acid and the copolymers thereof, polymers on the basis of (meth)acrylate, polyacrylates, polyisobutylenes, polyterpenes, ethylenevinyl acetate copolymers, hot-melt adhesives and substituted celluloses, preferably methyl and ethyl celluloses, and mixtures of these polymers; and that the transdermal therapeutic system contains
- a component from the group of the pyrrole derivatives which is 2-pyrrolidone, and
- limonene or a mixture of natural terpenes the main component of which is limonene.

2. Transdermal therapeutic system according to claim 1, **characterised in that** the said component from the group of the pyrrole derivatives is used in a concentration range between 0.1-15%, preferably from 2 to 10%, and said limonene or the said mixture of natural terpenes is used in a concentration range of 1-30%, preferably from 10-20%, all of these values being relative to the total mass of the matrix.

3. Transdermal therapeutic system according to claim 1 or 2, **characterised in that** at least one matrix layer contains esters of hydrogenated colophony, preferably its methyl or glycerol esters.

4. Transdermal therapeutic system according to one or more of the preceding claims, **characterised in that** it contains one or more plasticizers selected from the group comprising hydrocarbons, alcohols, carboxylic acids and their derivatives, ethers, esters or amines.

5. Transdermal therapeutic system according to claim 4, **characterised in that** it contains plasticizers in a concentration of 0-30%, preferably of 5-20%, all of these values being relative to the total mass of the matrix.

6. Transdermal therapeutic system according to one or more of the preceding claims, **characterised in that** it contains a natural or partially synthetic triglyceride or a mixture of these triglycerides in a concentration range of 0-30%, preferably in the range of 5-20%, relative to the total mass of the matrix.

7. Transdermal therapeutic system according to one or more of the preceding claims, **characterised in that** it contains at least one permeation accelerator selected from the group comprising polyoxyethylene derivatives and non-ionogenic tensides, preferably sorbitan fatty acid ester, the concentration of the permeation accelerator or accelerators being in the range of 0-5%, preferably of 1-3% relative to the total weight of the matrix.

8. Transdermal therapeutic system according to one or more of the preceding claims, **characterized in that** the total concentration of acetylsalicylic acid and/or salicylic acid is 5-75%, preferably 15-45%, relative to the total weight of the active substance-containing layers.

9. Transdermal therapeutic system in form of a patch, containing acetylsalicylic acid and/or salicylic acid, with a back layer, an active substance reservoir connected therewith, a membrane controlling the release of active substance if no other control mechanisms are present, a pressure-sensitive adhesive device for fixing the system to the skin and a protective layer to be separated before application, **characterized in that** it has a bag-shaped active substance reservoir filled with a liquid, highly viscous, semi-solid or thixotropic matrix, and that it contains
- a component from the group of the pyrrole derivatives which is 2-pyrrolidone, and
- limonene or a mixture of natural terpenes the main component of which is limonene.

10. Transdermal therapeutic system according to claim 9, **characterised in that** the bag-shaped active substance reservoir contains a gelatinizing agent.

11. Process for the production of a transdermal therapeutic system according to any one of claims 1 to 8, **characterised by** the following steps:
a) preparing a first polymer matrix layer containing 2-pyrrolidone and the said active substance, by coating a film with a suspension containing polyacrylate adhesive, 2-pyrrolidone and acetylsalicylic acid and/or salicylic acid, and subsequent drying;
b) preparing a second polymer matrix layer containing limonene, by coating a film with a solution which contains limonene or a mixture of natural terpenes the main component of which is limonene, and which also contains polymethacrylate;
c) forming a laminate comprising at least two layers, by laminating the dried, first polymer matrix layer to the still moist second polymer matrix layer.

## Revendications

1. Système thérapeutique transdermique sous forme d'emplâtre contenant de l'acide acétylsalicylique et/ou de l'acide salicylique, comprenant une couche dorsale, un réservoir pour principe actif qui y est relié, une membrane contrôlant la délivrance du principe actif en l'absence d'autres mécanismes de contrôle, un dispositif autoadhésif pour la fixation du système sur la peau et une couche de protection qui peut être enlevée avant l'application, **caractérisé en ce qu****'**il présente une structure stratifiée comprenant au moins une couche matricielle polymère, la ou les couches matricielles polymères étant constituées par des polymères qui sont choisis parmi le groupe comprenant des esters d'acides polyacryliques et leurs copolymères, des polymères à base de (méth)acrylates, des polyacrylates, des polyisobutylènes, des polyterpènes, des copolymères d'éthylène-acétate de vinyle, des adhésifs thermofusibles et des celluloses substituées, de préférence la méthylcellulose et l'éthylcellulose, et des mélanges de ces polymères ; et en ce que le système thérapeutique transdermique contient
- un constituant choisi parmi le groupe des dérivés du pyrrole, auquel cas il s'agit de la 2-pyrrolidone, et
- du limonène ou un mélange de terpènes naturels, dont le constituant principal est le limonène.

2. Système thérapeutique transdermique selon la revendication 1, **caractérisé en ce que** le constituant choisi parmi le groupe des dérivés du pyrrole est mis en oeuvre dans une plage de concentration de 0,1 à 15 %, de préférence de 2 à 10 %, et le limonène ou le mélange de terpènes naturels est mis en oeuvre dans une plage de concentration de 1 à 30 %, de préférence de 10 à 20 %, chaque fois rapportés à la masse totale de la matrice.

3. Système thérapeutique transdermique selon la revendication 1 ou 2, **caractérisé en ce que** au moins une couche matricielle contient des esters du colophane hydrogéné, de préférence son ester méthylique ou son ester de glycérol.

4. Système thérapeutique transdermique selon l'une quelconque des revendications précédentes, **caractérisé en ce qu'**il contient un ou plusieurs plastifiants choisis parmi le groupe qui contient des hydrocarbures, des alcools, des acides carboxyliques et leurs dérivés, des éthers, des esters ou des amines.

5. Système thérapeutique transdermique selon la revendication 4, **caractérisé en ce qu'**il contient des plastifiants en une concentration de 0 à 30 %, de préférence de 5 à 20 %, chaque fois rapportés à la masse totale de la matrice.

6. Système thérapeutique transdermique selon l'une quelconque des revendications précédentes, **caractérisé en ce qu****'**il contient un triglycéride naturel ou partiellement synthétique ou un mélange de ces triglycérides dans une plage de concentration de 0 à 30 %, de préférence dans la plage de 5 à 20 %, rapportés à la masse totale de la matrice.

7. Système thérapeutique transdermique selon l'une quelconque des revendications précédentes, **caractérisé en ce qu'**il contient au moins un accélérateur de la perméation choisi parmi le groupe qui comprend des dérivés du polyoxyéthylène et des agents tensioactifs non ionogènes, de préférence des esters d'acides gras de sorbitanne, la concentration du ou des accélérateurs de la perméation se situant dans la plage de 0 à 5 %, de préférence de 1 à 3 %, rapportés à la masse totale de la matrice.

8. Système thérapeutique transdermique selon l'une quelconque des revendications précédentes, **caractérisé en ce que** la concentration de l'acide acétylsalicylique et/ou de l'acide salicylique s'élève au total de 5 à 75 %, de préférence de 15 à 45 %, rapportés à la masse totale des couches contenant le principe actif.

9. Système thérapeutique transdermique sous forme d'emplâtre contenant de l'acide acétylsalicylique et/ou de l'acide salicylique, comprenant une couche dorsale, un réservoir pour principe actif qui y est relié, une membrane contrôlant la délivrance du principe actif en l'absence d'autres mécanismes de contrôle, un dispositif autoadhésif pour la fixation du système sur la peau et une couche de protection qui peut être enlevée avant l'application, **caractérisé en ce qu'**il possède un réservoir pour principe actif en forme de poche, rempli avec une matrice liquide, fortement visqueuse, semi-solide ou thixotrope, et contient
- un constituant choisi parmi le groupe des dérivés du pyrrole, auquel cas il s'agit de la 2-pyrrolidone, et
- du limonène ou un mélange de terpènes naturels, dont le constituant principal est le limonène.

10. Système thérapeutique transdermique selon la revendication 9, **caractérisé en ce que** le réservoir pour principe actif en forme de poche contient un gélifiant.

11. Procédé pour la préparation d'un système thérapeutique transdermique selon l'une quelconque des revendications 1 à 8, **caractérisé par** les étapes suivantes :
a) préparation d'une première couche matricielle polymère qui contient de la 2-pyrrolidone et le principe actif mentionné, par revêtement d'une feuille avec une suspension contenant un adhésif à base de polyacrylate, de la 2-pyrrolidone et de l'acide acétylsalicylique et/ou de l'acide salicylique, et par séchage ultérieur ;
b) préparation d'une deuxième couche matricielle polymère qui contient du limonène, par revêtement d'une feuille avec une solution contenant du limonène ou un mélange de terpènes naturels, dont le constituant principal est le limonène, ainsi que du polyméthacrylate ;
c) formation d'un stratifié comprenant au moins deux couches par application par contre-collage de la première couche matricielle polymère séchée sur la deuxième couche matricielle polymère encore humide.
